# FASCICULE DE BREVET EUROPEEN

(11) **EP 2 566 433 B1**
(45) Date de publication et mention de la délivrance du brevet: **28.08.2019**
(21) Numéro de dépôt: 09785822.9
(22) Date de dépôt: 23.02.2009
(51) Int. Cl.: A61K 6/027, C03C 4/00

(54) **NOUVELLE UTILISATION DE L'OPALE EN CHIRURGIE DENTAIRE ET LES COMPOSITIONS DESTINÉES A CETTE UTILISATION**
NEUE VERWENDUNG VON OPAL IN DER ZAHNCHIRURGIE UND ZUSAMMENSETZUNGEN DAFÜR
NOVEL USE OF OPAL IN DENTAL SURGERY AND COMPOSITIONS INTENDED FOR SAME

(43) Date de publication de la demande: 13.03.2013
(73) Titulaire: Patrinove, 69006 Lyon (FR)
(72) Inventeur: GUTIERREZ, Gilles, 69006 Lyon (FR); VLADILA, Bogdan, 050723 Bucarest (RO)
(74) Mandataire: Tripoz, Inès
(86) Numéro de dépôt international: PCT/IB2009/000388
(87) Numéro de publication internationale: WO 2010/094994

(56) Documents cités:
- WO-A1-2004/071327
- AU-B2- 696 562
- FR-A- 2 919 498
- FR-A- 2 926 460
- US-A1- 2005 175 552

## Description

La présente invention s'adresse aux nécessités de la vie et en particulier à la chirurgie dentaire. L'invention s'adresse en particulier aux problèmes de solidité de la dentition et de stabilité d'occlusion en augmentant la quantité de matière de contention à l'entour de la racine, d'une manière sûre et efficace.

La présente invention se propose donc de résoudre le problème de la mobilité dentaire ou de faiblesse d'intégration d'implant par une méthode basée sur la migration des cellules depuis l'os sain jusqu'à l'endroit du déficit cellulaire responsable de la mobilité. Les cellules migrent au sein de la matrice extracellulaire. C'est l'un des processus nécessaires pour immobiliser la dent ou la prothèse (implant):
La première étape consiste à rétablir la population des cellules puis ultérieurement à reconstruire la matrice extracellulaire et les ligaments des alvéoles dentaires. Cette matrice extracellulaire nouvellement formée maintient les cellules en place et constitue un matériau de contention.

Il a été constaté, que la mobilité des dents affecte un grand nombre de patients de tout âge mais que la mobilité augmente avec l'âge. Cette mobilité dentaire est augmentée par les infections microbiennes opportunistes, elle dégrade le confort du malade et réduit son espérance de vie. La mobilité dentaire gène la mastication. Elle est actuellement réduite par des techniques chirurgicales difficiles et lourdes avec des résultats faibles et peu satisfaisants. En effet, la mise en place d'une prothèse n'arrête pas le processus de destruction gingivale et favorise l'accumulation bactérienne induite par la rétention des aliments. Le blocage se fait en ayant recours aux implants dentaires.
Les poches parodontales profondes sont associées à la mobilité dentaire. La migration cellulaire provoque une réduction des poches parodontales.

Si on désire immobiliser une dent ou un pilier, cela nécessite de resserrer la racine de la dent ou de l'implant dans la mâchoire. Il faut pour cela accroître la quantité de matière à l'entour de la base de la dent aux fins de la resserrer mécaniquement dans l'os. De plus l'apport de cellules va permettre la reconstruction des ligaments alvéolos-dentaires détruits par sénescence, infection (nécrose), par un traumatisme ou par un processus dégénératif. De plus ce matériel permet de mieux structurer l'interface os-implant.

### I Etat antérieur de l'art

Il a été montré que l'opale accélère la migration des cellules. (Voir demande de brevet français n° 07- 04336 déposé le 18/06/2007). Les demandeurs ont montré que le silicate, matière de l'opale est organisé en sphères agencées en plan. Lors de la synthèse en laboratoire, les sphères sont parfois agencées comme dans l'état naturel; le plus souvent les sphères sont associées sous forme d'agglomérats. Ces agglomérats de sphères peuvent sous certaines conditions émettre un signal attractif des cellules eucaryotes. Les auteurs ont de plus constaté que le signal émis par l'opale traverse un certain nombre de matériaux comme les céramiques (dérivés de l'alumine) et le zircon pouvant être employé comme matériau de prothèse, convenait mieux à l'utilisation de l'opale que les métaux.

Lorsqu'une dent devient mobile, le nerf est très rapidement lésé car le pertuis à travers lequel, il passe de la pulpe à l'os à travers la racine est extraordinairement fin. Le nerf, à ce niveau de la racine passe à travers un trou de quelques dizaines de microns. On conçoit aisément qu'un mouvement de quelques millimètres créé un effet de cisaillement suffisant pour provoquer la rupture du nerf lui-même. L'ablation de la pulpe et de son nerf devient rapidement indispensable pour désensibiliser la dent. L'ouverture d'un pertuis au sein de l'émail jusqu'à la pulpe rends aisé l'accès à cette cavité naturelle ou artificiellement ouverte. Il est alors possible d'envisager d'y introduire de l'opale au sein de cette cavité aux fins de provoquer une migration des cellules vers la racine. La mise en place d'opale dans le canal dentaire en remplacement de la pulpe et du nerf est malaisée. En effet, le canal dentaire est irrégulier et souvent il est impossible de le rendre rectiligne. Son diamètre varie de quelques dizaines de microns à guère plus d'un millimètre. Le réalésage des canaux ne peut pas garantir une étanchéité d'obturation canalaire. La manipulation est difficile aussi en raison d'écoulement incertain de la poudre. La poudre pourrait être avantageusement mélangée à un liquide. L'écoulement d'un liquide dans le canal pose d'emblée le problème de la rhéologie de l'introduction et le contrôle de la dose introduite. L'association avec des cônes de cire ou de gutta peut être envisagée mais la chose est malaisée car la matière est friable et peut se briser avant d'atteindre le fond, ce qui est préjudiciable à l'obtention d'un bon résultat.

C'est pourquoi, l'opale doit pouvoir être introduite sous une forme appropriée aux fins d'atteindre l'ensemble du canal dentaire, c'est-à-dire pénétrer aussi profondément que possible dans le canal et obstruer ainsi autant la partie la plus étroite que la partie la plus évasée.

Une situation similaire se présente lorsque le chirurgien met en place un implant dentaire. La base destinée à recevoir l'implant, présente toujours une cavité destinée aux connexions prothétiques qui sont mises en place quatre mois plus tard. Ces 4 mois (période d'intégration d'implant) pourront être réduits considérablement en incorporant dans la cavité précitée un polymère contenant de l'opale tel que décrit plus loin .

Cette forme d'utilisation de l'opale, objet de l'invention doit présenter les avantages suivants pour le praticien :
a) Stérilisation facile.
b) Innocuité.
c) Maintien des propriétés de l'opale.
d) Résistance aux solvants usuels
e) Facilité de manipulation, d'adaptation à la cavité dentaire et d'introduction dans le canal dentaire
f) Possibilité de retrait.
g) Possibilité de garantir une étanchéité d'obturation.

Dans l'état antérieur de l'art, le remplissage de la cavité destinée à recevoir les connexions prothétiques était obstrué à l'aide d'une vis de cicatrisation. Le raccourcissement de celle-ci ou son absence laissera de la place pour insérer le polymère contenant l'opale. De même le remplissage du canal dentaire après son évidement, se fait à l'aide de cônes, de cires ou de gutta. Les cires thermo fusibles présentent de nombreux avantages car qu'elles soient constituées d'esters du glycérol ou d'éthers d'alcool à longue chaîne, elles sont malléables et leurs propriétés physico-chimiques tout comme leur biocompatibilité sont bien connues. Un autre avantage des cires réside dans la possibilité de former des éléments de forme conique ou cylindrique convenant à l'introduction dans le canal dentaire et de les compacter. L'inconvénient des cires est dû au fait que soit, elles se cassent lors de l'introduction dans le canal, soit si elles sont trop souples, elles transmettent mal le signal généré par les agglomérats d'opale. L'utilisation de la gutta est aussi très appropriée en ce qui concerne le remplissage du canal mais elle présente l'inconvénient d'étouffer le signal des particules d'opale introduites en son sein. Dans le document WO2004/071327 sont décrits des gels comme la gutta et le polybutadiène destinés à limiter les problèmes d'accroche et d'adhérence entre le matériau de comblement et les plaquettes des obturateurs.

Ces propriétés suggèrent l'utilisation de polymères plastiques thermo-fusibles de type polyéthylène ou polypropylène dont les températures de fusion sont de l'ordre de 100°C. Ils ont aussi un historique bien établi en tant que biomatériaux implantés dans le corps humain. Leur éventuelle dégradation se fait sans libérer de débris nocifs. Leur résistance sans déformation permet leur introduction dans le canal et l'extraction éventuelle hors du canal dentaire du matériel mis en place par le praticien. Leur température de fusion permet de travailler le produit en bouche.

L'invention consiste donc dans l'utilisation d'une composition à base d'opale synthétique ou naturelle incorporée dans un polymère facilement fusible sous forme de fibres cylindriques ou de rubans destinés à être insérés dans le canal dentaire.

Plus particulièrement, l'invention concerne une composition à base d'opale synthétique ou naturelle incorporée dans un polymère choisi parmi le polyéthylène, le polypropylène, les acrylates ou les méthacrylates facilement fusible sous forme de fibres cylindriques ou de rubans pour une utilisation dans la cavité canalaire d'une dent naturelle ou de cavités créées lors d'implants dans une dent ou un implant.

Selon un mode de réalisation, la composition est utilisée pour réduire la mobilité d'une dent ou améliorer l'intégration d'un implant.

Le polymère facilement fusible est de préférence le polyéthylène et principalement le polyéthylène haute densité (HDPE), le poly éthylène moyenne densité (MDPE) ou encore le polyéthylène basse densité (BDPE). Le polypropylène également fusible convient d'une manière similaire ainsi que les polyacrylates et les polyméthacrylates. Les polyacrylates et les polyméthacrylates présentent en outre la particularité d'être liquides à l'état de monomère ou d'oligomère et il est possible de disperser dans ce liquide la quantité appropriée de poudre d'opale (de 0,5 à 20 % et de préférence de 1 à 15 % en poids) puis d'effectuer la polymérisation du monomère selon les méthodes classiques de polymérisation. Il est d'usage courant en art dentaire de photopolymériser ces résines avec des photons de lumière violette à ultraviolette (150 à 300 nm)

Selon un mode de réalisation, la composition est caractérisée en ce qu'elle comprend de 0,5% à 20% en poids d'opale naturelle ou synthétique.

Selon des modalités particulières de l'invention il a été possible d'incorporer jusqu'à 20 % d'opale dans le polyéthylène (PE) en fusion à 110°C. Les variétés HDPE, MDPE et BDPE ont donné des résultats assez similaires. La poudre d'opale est caractérisée par le fait qu'elle est composée de sphères dont le diamètre est compris entre 150 et 600 mn agglomérées en amas de taille comprise entre 0,500 et 3,00 µm. Les résultats sur la vitesse de migration montrent une meilleure efficacité lorsqu'on utilise des sphères dont le diamètre est compris entre 200 et 500 nm formant des amas de tailles comprises entre 0,5 et 1,5 µm. Des essais ont permis d'obtenir des résultats satisfaisants avec des matériaux comme les polypropylènes contrairement au polybutadiène qui étouffe sensiblement le signal tout comme la gutta.

Les fibres de polyamides sont trop souples pour être introduits dans le canal.

Les demandeurs ont déterminé que les concentrations de 10 % en opale sont préférables pour une meilleure préservation des propriétés du polymère permettant d'optimiser la mise en place intra canalaire.

Les demandeurs ont obtenu en outre des résultats satisfaisants avec les polyacrylates, notamment le polyméthacrylate de méthyle. La solution d'oligomère de ce polymère peut incorporer, comme les polyéthylènes ou polypropylènes de 0,5 à 20 % et de préférence de 2 à 10 % de poudre d'opale. La polymérisation se fait aisément avec un initiateur physique UV ou chimique comme un peroxyde ou tout autre agent de polymérisation associé ou non avec un agent réticulant. L'acrylate peut être utilisé pour des racines très minces (0,1- 0,2 mm) parce qu'il est peu flexible et pourra pénétrer le plus profondément dans les canaux. Après avoir inséré le premier cône acrylique en profondeur, on pourra compacter des cônes flexibles de polyamides ou autres pour obtenir une étanchéité d'obturation.

Le matériel testé se caractérise en ce qu'il se présente soit sous forme de fibres cylindriques de diamètre compris entre 0,3 et 1,5 mm, soit sous forme cylindrique de diamètre compris entre 0,3 et 1,5 mm soit sous forme de mince ruban de 0,3 mm d'épaisseur et de 0,3 à 1 mm de largeur. La longueur de ces matériaux est peu déterminante car seule la partie introduite au plus profond de la cavité de la dent sera efficace, l'excès de matière contenant l'opale pourra être sectionné à l'aide de l'instrument adéquat.

Des essais en culture cellulaire montrent que si la migration des fibroblastes de peau humaine est fortement accélérée, puisqu'elle passe à titre d'exemple de 3 µm/h à plus de 8 µm/h, des résultats similaires sont obtenus avec des cémentoblastes, des ostéoblastes et des kératinocytes, notamment des kératinocytes gingivaux et même des kératinocytes issus d'explants de peau humaine.

Ces cellules ont toujours des vitesses de migration plus faibles de l'ordre de quelques microns par heure quand elles ne sont pas soumises au signal issu du polymère contenant 10 % d'opale. En présence des compositions contenant de l'opale, les demandeurs ont observé un accroissement de la vitesse de migration le plus souvent compris entre 50 % et 125 %.

La mise en place de 2 à 6 éléments en polyéthylène HDPE dans les canaux dentaires des molaires tout comme dans le canal des canines ou des incisives est très aisée. La mobilité dentaire est réduite en moyenne de 30 à 50 % en quelques semaines. La vitesse d'obtention des résultats cliniques dépend de la taille du canal et donc de la quantité d'opale pouvant être introduite dans le canal. La taille de la dent joue ainsi un rôle important. Plus les racines offrent un diamètre extérieur important plus le résultat est obtenu rapidement car le recrutement cellulaire est plus important. De même, le remplissage d'une cavité d'une prothèse dépend de la quantité de polymère utilisé. La quantité d'opale est très importante, car la vitesse de migration (et donc l'efficacité) est directement proportionnelle à la quantité de cellules progénitrices pouvant être recrutées aux environs de ligaments alvéolodentaires ou implant inséré. Ce recrutement ou la dimension du diamètre formé par la population de cellules recrutées est directement fonction de la quantité de signal émis et donc de la quantité d'opale présente dans le canal. L'intensité du signal dépend donc de la quantité d'opale disponible.

La diminution de la mobilité dentaire se traduit aussi sur le plan clinique par l'amélioration de l'occlusion de la mâchoire et ainsi par une meilleure efficacité de la mastication car le patient aura une position mandibulaire reproductible pour une occlusion adéquate et les muscles masticatoires seront moins sollicités et donc moins fatigués en essayent de trouver la position de force masticatoire maximale. De même la meilleure intégration de l'implant permettra une mise en charge plus rapide et une possible réparation d'une périmplantitis.

Pour l'utilisation avec une gouttière plastique plaquée avec de l'Opale (méthode de prévention) on réalisera des bandes flexibles de 0,4 mm de largeur et 0,3 mm de hauteur et dont la longueur est déterminée objectivement par un appareil destiné à cet usage qui va les coller sur les bords externes et internes des gouttières, correspondant à l'épithélium de jonction qui deviendra plus résistent contre les agressions externes (microbiennes ou traumatiques). Cette méthode ne demande aucune action de soin dentaire, seulement une banale prise d'emprunt et est recommandée pour tous les patients afin de renforcer préventivement la gencive naturelle durant la nuit qui permet ainsi une intégration d'implant plus rapide et d'une meilleure qualité car les cellules progénitrices arriveront au niveau d'interface implant-os avant le plongement de cellules épithéliales. Une autre utilisation concerne le traitement des periimplantitis par attraction des cellules progénitrices au niveau des zones altérées. Cela sera réalisé par l'introduction de l'Opale l'intérieur de l'implant, dans le trou existant pour recevoir les éléments prothétiques ou par l'utilisation de gouttières plaquées avec Opale.

## Revendications

1. Composition à base d'opale synthétique ou naturelle incorporée dans un polymère choisi parmi le polyéthylène, le polypropylène, les acrylates ou les méthacrylates facilement fusible sous forme de fibres cylindriques ou de rubans pour une utilisation dans la cavité canalaire d'une dent naturelle ou de cavités créées lors d'implants dans une dent ou un implant.

2. Composition selon la revendication 1, pour réduire la mobilité d'une dent ou améliorer l'intégration d'un implant.

3. Composition selon l'une quelconque des revendications 1 à 2, **caractérisée en ce qu'**elle comprend de 0,5 % à 20 % en poids d'opale naturelle ou synthétique.

## Patentansprüche

1. Zusammensetzung auf der Grundlage von synthetischem oder natürlichem, in einem Polymer enthaltenem Opal, wobei das Polymer ausgewählt ist aus Polyethylen, Polypropylen, Acrylaten oder Methacrylaten, die als zylindrische Fasern oder Band leicht schmelzbar sind, zur Verwendung in dem kanalförmigen Hohlraum eines natürlichen Zahns oder bei Implantaten erzeugten Hohlräumen in einem Zahn oder einem Implantat.

2. Zusammensetzung nach Anspruch 1, zum Verringern der Beweglichkeit eines Zahns oder zum Verbessern der Integration eines Implantats.

3. Zusammensetzung nach einem der Ansprüche 1 bis 2, **dadurch gekennzeichnet, dass** sie 0,5 bis 20 Gew.-% natürlichen oder synthetischen Opal umfasst.

## Claims

1. A composition based on synthetic or natural opal incorporated in a polymer selected from polyethylene, polypropylene, acrylates or methacrylates, which is readily meltable, in the form of cylindrical fibers or of ribbons for use in the canal cavity of a natural tooth or in cavities created in the case of implants in a tooth or an implant.

2. The composition according to Claim 1, for reducing the mobility of a tooth or improving the integration of an implant.

3. The composition according to any one of Claims 1 to 2, **characterized in that** it comprises 0.5% to 20% by weight of natural or synthetic opal.
